# EUROPEAN PATENT APPLICATION

(11) **EP 4 019 544 A1**
(43) Date of publication of application: **29.06.2022**
(21) Application number: 20854930.3
(22) Date of filing: 11.08.2020
(51) Int. Cl.: C07K 16/18, C07K 16/28, C07K 16/30, C07K 16/32, A61K 47/68, A61K 51/10

(54) **ANTIBODY MUTANT AND APPLICATION THEREOF**

(30) Priority: 19.08.2019 CN 201910766943
(71) Applicant: Shenyang Pharmaceutical University, Shenyang Liaoning 110016 (CN); Beijing Sipuruige Biotech Corporation, Beijing 100176 (CN)
(72) Inventor: MA, Ningning, Shenyang, Liaoning 110016 (CN); WANG, Lin, Shenyang, Liaoning 110016 (CN); SONG, Chunyu, Beijing 102600 (CN); LI, Mingying, Benxi, Liaoning 117004 (CN); XUE, Shiping, Province 037006 (CN); LIU, Yongxiang, Shenyang, Liaoning 110016 (CN); XU, Weiwei, Beijing 102600 (CN); ZHU, Lifeng, Shijiazhuang, Hebei 050035 (CN)
(74) Representative: Nederlandsch Octrooibureau
(86) International application number: PCT/CN2020/108434
(87) International publication number: WO 2021/031930

(57) **Abstract**

Provided is a mutant of an antibody or fragment thereof, characterized in that the antibody or fragment thereof contains a light-chain constant region, and according to the Kabat numbering system, the amino acid at position 166 is mutated to cysteine.

## Description

### FIELD OF THE INVENTION

The present invention relates to an antibody. Specifically, the present invention relates to an antibody mutant, a composition and/or conjugate containing the antibody mutant, and use thereof.

### BACKGROUND OF THE INVENTION

Antibody-drug conjugates (ADCs) are targeted therapeutic drugs that combine the specificity of antibodies with the cytotoxicity of cytotoxic therapeutic agents. ADCs are mainly considered as candidates for the treatment of various cancers. ADCs contain antibodies linked to therapeutic drugs. In addition, bispecific antibodies can also be synthesized quickly and in large quantities from two existing antibodies by chemical coupling. For example, two IgG molecules or two Fab fragments are linked by a coupling reagent. It is also reported to prepare CovX-Bodies by using an abzyme [1], wherein two polypeptides with target neutralization effects are linked to two Fab arms of one IgG molecule respectively. Therefore, site-directed coupling technology can also be applied to the preparation of novel bispecific antibodies [1].

Antibody molecules contain many groups that can be used for modification or crosslinking, such as amino group and carboxyl group. However, the large number of these groups in the antibody molecule results in the randomness of antibody coupling site. There are also many methods that rely on disulfide bonds on antibody molecules, wherein since the number of disulfide bond is limited and the position of disulfide bond is relatively fixed, the antigen-binding site of antibody will not be shielded after the drug is coupled. These characteristics make the disulfide bond on the antibody become one of the suitable sites for antibody coupling. However, the reduction of these endogenous disulfide bonds is non-specific, that is, the disulfide bonds in the hinge region and the disulfide bonds between the heavy and light chains are all reduced, resulting in the coupling product being a mixture of diverse components [2,3].

Cysteine thiols are reactive at neutral pH, which is different from most amines whose protonation and nucleophilicity decrease at near pH7. Due to the relative reactivity of free thiols (sulfhydryl groups), proteins with cysteine residues usually exist in oxidized form as disulfide-linked oligomers or have internally bridged disulfide group. Cysteine sulfhydryl groups in antibodies are generally more reactive to electrophilic coupling reagents than antibody amines or hydroxyl groups, that is, more nucleophilic. Therefore, the amino acid residues in the antibody can be mutated to a cysteine, and then the cysteine can be used for conjugation reaction. Junutula et al. [4] researched and developed a PHESELECTOR (phage ELISA for selection of reactive thiols) technology, and this technology was applied to screen and obtain an IgG mutant THIOMAB containing reactive Cys. The product obtained after coupling the IgG mutant THIOMAB with drug is called TDC (THIOMAB-drug conjugates). Compared with conventional ADCs, the corresponding TDC obtained by PHESELECTOR technology shows better therapeutic effects, but there are also drawbacks: when a full-length antibody is expressed in a mammalian cell, the introduced Cys can form a disulfide bond with a glutathione or other sulfhydryl-containing substance in the culture medium, so the introduced Cys needs to be reduced into a free form for coupling, but meanwhile, the interchain disulfide bond of the antibody will also be opened, which needs to be oxidized again to restore to be a complete antibody; and this additionally introduced sulfhydryl group may also mistakenly form disulfide bonds between the two Fabs of the antibody during the whole process of THIOMAB formation[5-7].

When appropriate sites are selected for cysteine mutation, the sulfhydryl group on the cysteine obtained by mutation can always remain in a free state during the expression and purification process, and can be directly applied to downstream coupling reactions without any pretreatment. Therefore, the applicant has developed reasonable cysteine mutation sites, and the cysteine sulfhydryl groups obtained after mutations at these sites are easier to maintain a free state and have higher reactivity. Afterwards, the antibody is coupled to another functional molecule (an antibody fragment, polypeptide or small molecule drug) through a maleimide-containing linker.

### SUMMARY OF THE INVENTION

The present invention mainly provides a site that can be mutated to a cysteine in a constant region of an antibody, wherein the mutation introduces a selected site-specific conjugation site to allow the antibody, fragment or derivative thereof to be conjugated with an effective loading, wherein the one or more mutations include a mutation at position 166 of a light chain (according to Kabat numbering system). Meanwhile, the present invention also provides an independent structure of a bispecific antibody with anti-angiogenesis function.

Therefore, the present invention provides the following aspects:
1. A variant of antibody or fragment thereof, wherein the antibody or fragment thereof comprises a light chain constant region, and an amino acid at position 166 according to a Kabat numbering system is mutated to cysteine, preferably, the antibody is a humanized antibody, a chimeric antibody, more preferably an IgG antibody.
2. The variant of antibody or fragment thereof of 1, wherein the light chain is of a lambda or kappa type.
3. The variant of antibody or fragment thereof of 1 or 2, which comprises a heavy chain and a light chain, wherein an amino acid sequence of the heavy chain is shown in SEQ ID NO: 1, and an amino acid sequence of the light chain is shown in SEQ ID NO: 7; the amino acid sequence of the heavy chain is shown in SEQ ID NO: 9, and the amino acid sequence of the light chain is shown in SEQ ID NO: 13; or the amino acid sequence of the heavy chain is shown in SEQ ID NO : 17, and the amino acid sequence of the light chain is shown in SEQ ID NO:21.
4. The variant of antibody or fragment thereof of any one of 1 to 3, wherein the fragment is a Fab fragment, a Fab' fragment or a F(ab')₂ fragment, preferably a heavy chain amino acid sequence of the Fab fragment is shown in SEQ ID NO: 25, and a light chain amino acid sequence of the Fab fragment is shown in SEQ ID NO: 29.
5. A bispecific antibody or fusion protein, comprising the variant of antibody or fragment thereof of any one of 1 to 4.
6. A conjugate, comprising the variant of antibody or fragment thereof of any one of 1 to 4.
7. The conjugate of 6, wherein the conjugate is selected from polyethylene glycol, a cytotoxic agent, an active peptide, an nanobody, a single domain antibody, a Fab fragment, a Fab' fragment, scFv, a small molecule drug, a chemotherapeutic agent or a radiotherapeutic agent; preferably, the active peptide is an Agn2 active peptide, more preferably an Agn2 active peptide as shown in SEQ ID NO: 5; more preferably, the Agn2 active peptide is conjugated to a bevacizumab variant of the present invention via a linker; further preferably, the linker has an amide bond, specifically is formamide-PEG₄-NHS with a structure as shown in formula I 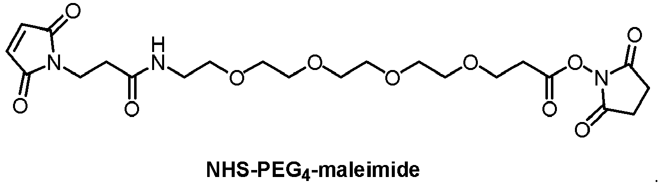
8. The conjugate of 6, wherein the polyethylene glycol is monomethoxy polyethylene glycol, preferably mPEG2000, mPEG5000, mPEG10000.
9. The conjugate of 7 or 8, wherein the polyethylene glycol is further conjugated to a drug molecule, preferably the polyethylene glycol is a linear difunctionalized polyethylene glycol, a linear heterofunctionalized polyethylene glycol or a multi-arm functionalized polyethylene glycol.
10. A pharmaceutical composition comprising the variant of antibody or fragment thereof of any one of 1 to 4, the bispecific antibody or fusion protein of 5, the conjugate of any one of 6 to 9, and optionally, a pharmaceutically acceptable carrier.
11. A method for treatment of cancer (preferably non-small cell lung cancer such as advanced, metastatic or recurrent non-squamous cell non-small cell lung cancer, colorectal cancer such as metastatic colorectal cancer, breast cancer, malignant glioma and renal cell carcinoma, glioblastoma multiforme), comprising administering the variant of antibody or fragment thereof of any one of 1 to 4, the bispecific antibody or fusion protein of 5, the conjugate of any one of 6 to 9, or the pharmaceutical composition of 10.
12. Use of the variant of antibody or fragment thereof of any one of 1 to 4, the bispecific antibody or fusion protein of 5, or the conjugate of any one of 6 to 9, or the pharmaceutical composition of 10 in the preparation of a medicament or kit for treatment of cancer (preferably non-small cell lung cancer such as advanced, metastatic or recurrent non-squamous cell non-small cell lung cancer, colorectal cancer such as metastatic colorectal cancer, breast cancer, malignant glioma and renal cell carcinoma, glioblastoma multiforme).
13. A kit comprising the variant of antibody or fragment thereof of any one of 1 to 4, the bispecific antibody/fusion protein of 5, or the conjugate of any one of 6 to 9, or the pharmaceutical composition of 10, preferably, the kit further comprises an agent to be administered in combination with the antibody or fragment thereof, such as carboplatin or cisplatin.

### BRIEF DESCRIPTION OF THE DRAWINGS

Fig.1 shows the SDS-PAGE electropherograms of wild-type (WT, also called "anti-VEGF antibody" in the present invention) antibody, 166 mutant antibody (166C), and 124 mutant antibody (124C) in a reduced and non-reduced state.
Fig.2 shows SDS-PAGE electrophoretograms of the anti-VEGF wild-type antibody and the antibody mutant after being coupled with mPEG2000-MAL. It can be seen from this figure that both 166C and 124C can be successfully coupled with the conjugate mPEG2000.
Fig.3 shows SDS-PAGE electrophoretograms of the anti-VEGF wild-type antibody and the antibody mutant after being coupled with DC (polypeptide-linker). By using gray scale analysis, it can be seen that 85% of 166C was successfully coupled to DC, while only 75% of 124C was successfully coupled to DC. It can be seen that under the same condition, the 166C antibody mutant showed better conjugation efficiency.
Fig.4 shows SDS-PAGE electrophoretograms of the anti-CD20 wild-type antibody and the antibody mutant after being coupled with mPEG2000-MAL.
Fig.5 shows SDS-PAGE electrophoretograms of the anti-CD20 wild-type antibody and the antibody mutant after being coupled with DC (polypeptide-linker).
Fig.6 shows SDS-PAGE electrophoretograms of the anti-HER2 wild-type antibody and the antibody mutant after being coupled with mPEG2000-MAL.
Fig.7 shows SDS-PAGE electrophoretograms of the anti-HER2 wild-type antibody and the antibody mutant after being coupled with DC (polypeptide-linker).
Fig.8 shows SDS-PAGE electrophoretograms of the anti-VEGF antibody Fab fragment wild-type antibody and the antibody mutant after being coupled with DC (polypeptide-linker).
Fig.9 shows SDS-PAGE electrophoretograms of the anti-VEGF antibody Fab fragment wild-type antibody and the antibody mutant after being coupled with mPEG2000-MAL.
Figure 10 shows LC/MS spectrum of the mutant 166C before coupling.
Figure 11 shows LC/MS spectrum of the mutant 166C after being coupled with DC.
Figure 12 shows LC/MS spectrum of the mutant 124C before coupling.
Figure 13 shows LC/MS spectrum of the mutant 124C after being coupled with DC.
Fig.14 shows inhibition of wild-type antibody (WT), antibody mutants (124C and 166C) and conjugates (124ADC and 166ADC) on the proliferation of endothelial cells HUVEC (proliferation produced by VEGF stimulation). It can be seen from the figure that the activity of the antibody mutant is not changed due to the cysteine mutation at the selected site, indicating that the selected site will not affect the biological activity of the antibody. Similarly, the conjugate generated by conjugating with DC does not affect the activity of the antibody to inhibit cell proliferation.

### Examples

In order to make the purpose, technical solution, and advantage of the present invention more clear, the present invention will be further described in detail below with reference to specific Examples. It will be understood by those skilled in the art that the following Examples are only for the purpose of illustration, and the protection scope of the present invention shall be based on the appended claims.

The restriction endonucleases used in the Examples were purchased from Thermo Fisher Scientific (China) Co., Ltd., and the reagents or materials used in the following Examples can be routinely purchased in the art unless otherwise specified. The culture medium of CHO-K1 cells was purchased from sigma.

### Example 1 Screening and expression of cysteine mutated cell lines

### (1) Construction of expression vector of anti-VEGF antibody and antibody mutant

The DNA encoding the antibody described herein (the corresponding wild-type sequence was obtained from USP Medicines Compendium, specially the heavy chain sequence is shown in SEQ ID NO: 1 and the light chain sequence is shown in SEQ ID NO: 2; based on this, according to kabat numbering, the amino acid at position 166 of the light chain was substituted with cysteine to obtain a variant 166C of the present invention, and the amino acid at position 124 of the light chain was substituted with cysteine to obtain a control antibody 124C) was chemically synthesized (Suzhou GENEWIZ Biotechnology Co., Ltd.), then the antibody heavy chain gene was digested with BstBI and PacI, and the light chain gene was digested with HindIII and EcoRI; T4 ligase was used to ligate the heavy chain gene to an eukaryotic expression vector pCGS3 (Biovector NTCC Inc.) treated by the BstBI and PacI; after successful ligation, the expression vector pCGS3 was digested with HindIII and EcoRI, and then the light chain gene was ligated to the expression vector pCGS3 by T4 ligase, that is, an expression vector containing both heavy and light chains was successfully constructed.

| | WT Bevacizumab | 166C | 124C |
|---|---|---|---|
| Encoding Sequence of Heavy Chain | SEQ ID NO:3 | SEQ ID NO:3 | SEQ ID NO:3 |
| Encoding Sequence of Light Chain | SEQ ID NO:4 | SEQ ID NO:5 | SEQ ID NO:6 |
| Amino Acid Sequence of Heavy Chain | SEQ ID NO:1 | SEQ ID NO:1 | SEQ ID NO: 1 |
| Amino Acid Sequence of Light Chain | SEQ ID NO:2 | SEQ ID NO:7 | SEQ ID NO:8 |

### (2) Screening and purification of expression vector of anti-VEGF antibody and antibody mutant

The successfully constructed expression vector was transformed into DH5α competent *E. coli* strain. The transformed DH5α strain was digested with BstBI and PacI, as well as HindIII and EcoRI respectively, and was verified by sequencing, and then positive clones were selected. A plasmid extraction kit (purchased from CoWin Biosciences) was used to lyse the resulting *E. coli* and to extract the plasmid, thereby obtaining an expression vector containing heavy and light chains.

### (3) Expression of anti-VEGF antibody and antibody mutant in CHO cells

The purified expression vector was transfected into CHO-K1 (purchased from ATCC) cells by electroporation; the cells were plated in a 96-well plate, grown for 15 days, and then single clones were picked out, and antibody yield of the cells was determined by ELISA. The first 20% cells were transferred into a 24-well plate. After 7 days of growth, the antibody yield of the cells was determined, and the first 5 to 6 strains were selected and transferred into a shake flask for culture, thereby achieving the expression of anti-VEGF antibody in CHO-K1 cells. After the serum-free culture was stable, the cells were continuously cultured for 7 days, and then collected for purification and follow-up experiments.

### (4) Construction of expression vector of anti-CD20 antibody and mutant

The DNA encoding the antibody described herein (the corresponding wild-type sequence was obtained from USP Medicines Compendium, specially the heavy chain sequence is shown in SEQ ID NO: 9 and the light chain sequence is shown in SEQ ID NO: 10; based on this, according to kabat numbering, the amino acid at position 166 of the light chain was substituted with cysteine to obtain a variant 166C of the present invention, and the amino acid at position 124 of the light chain was substituted with cysteine to obtain a control antibody 124C) was chemically synthesized (Suzhou GENEWIZ Biotechnology Co., Ltd.), then the antibody heavy chain gene was digested with BstBI and PacI, and the light chain gene was digested with HindIII and EcoRI; T4 ligase was used to ligate the heavy chain gene to an eukaryotic expression vector pCGS3 (Biovector NTCC Inc.) treated by the BstBI and PacI; after successful ligation, the expression vector pCGS3 was digested with HindIII and EcoRI, and then the light chain gene was ligated to the expression vector pCGS3 by T4 ligase, that is, an expression vector containing both heavy and light chains was successfully constructed.

| | WT Rituximab | 166C | 124C |
|---|---|---|---|
| Encoding Sequence of Heavy Chain | SEQ ID NO:11 | SEQ ID NO:11 | SEQ ID NO:11 |
| Encoding Sequence of Light Chain | SEQ ID NO:12 | SEQ ID NO:14 | SEQ ID NO:16 |
| Amino Acid Sequence of Heavy Chain | SEQ ID NO:9 | SEQ ID NO:9 | SEQ ID NO:9 |
| Amino Acid Sequence of Light Chain | SEQ ID NO: 10 | SEQ ID NO: 13 | SEQ ID NO:15 |

### (5) Screening and purification of expression vector of anti-CD20 antibody and antibody mutant

The successfully constructed expression vector was transformed into DH5α competent *E. coli* strain. The transformed DH5α strain was digested with BstBI and PacI, as well as HindIII and EcoRI respectively, and was verified by sequencing, and then positive clones were selected. A plasmid extraction kit (purchased from CoWin Biosciences) was used to lyse the resulting *E. coli* and to extract the plasmid, thereby obtaining an expression vector containing heavy and light chains.

### (6) Expression of anti-CD20 antibody and antibody mutant in CHO cells

The purified expression vector was transfected into CHO-K1 (purchased from ATCC) cells by electroporation; the cells were plated in a 96-well plate, grown for 15 days, and then single clones were picked out, and the antibody yield of the cells was determined by ELISA. The first 20% cells were transferred into a 24-well plate. After 7 days of growth, the antibody yield of the cells was determined, and the first 5 to 6 strains were selected and transferred into a shake flask for culture, thereby achieving the expression of anti-CD20 antibody in CHO-K1 cells. After the serum-free culture was stable, the cells were continuously cultured for 7 days, and then collected for purification and follow-up experiments.

### (7) Construction of expression vector of anti-HER2 antibody and antibody mutant

The DNA encoding the antibody described herein (the corresponding wild-type sequence was obtained from USP Medicines Compendium, specially the heavy chain sequence is shown in SEQ ID NO: 17 and the light chain sequence is shown in SEQ ID NO: 19; based on this, according to kabat numbering, the amino acid at position 166 of the light chain was substituted with cysteine to obtain a variant 166C of the present invention, and the amino acid at position 124 of the light chain was substituted with cysteine to obtain a control antibody 124C) was chemically synthesized (Suzhou GENEWIZ Biotechnology Co., Ltd.), then the antibody heavy chain gene was digested with BstBI and PacI, and the light chain gene was digested with HindIII and EcoRI; T4 ligase was used to ligate the heavy chain gene to an eukaryotic expression vector pCGS3 (Biovector NTCC Inc.) treated by the BstBI and PacI; after successful ligation, the expression vector pCGS3was digested with HindIII and EcoRI, and then the light chain gene was ligated to the expression vector pCGS3 by T4 ligase, that is, an expression vector containing both heavy and light chains was successfully constructed.

| | WT Trastuzumab | 166C | 124C |
|---|---|---|---|
| Encoding Sequence of Heavy Chain | SEQ ID NO:18 | SEQ ID NO:18 | SEQ ID NO:18 |
| Encoding Sequence of Light Chain | SEQ ID NO:20 | SEQ ID NO:22 | SEQ ID NO:24 |
| Amino Acid Sequence of Heavy Chain | SEQ ID NO:17 | SEQ ID NO:17 | SEQ ID NO:17 |
| Amino Acid Sequence of Light Chain | SEQ ID NO:19 | SEQ ID NO:21 | SEQ ID NO:23 |

### (8) Screening and purification of expression vector of anti-HER2 antibody and antibody mutant

The successfully constructed expression vector was transformed into DH5α competent *E. coli* strain. The transformed DH5α strain was digested with BstBI and PacI, as well as HindIII and EcoRI respectively, and was verified by sequencing, and then positive clones were selected. A plasmid extraction kit (purchased from CoWin Biosciences) was used to lyse the resulting *E. coli* and to extract the plasmid, thereby obtaining an expression vector containing heavy and light chains.

### (9) Expression of anti-HER2 antibody and antibody mutant in CHO cells

The purified expression vector was transfected into CHO-K1 (purchased from ATCC) cells by electroporation; the cells were plated in a 96-well plate, grown for 15 days, and then single clones were picked out, and the antibody yield of the cells was determined by ELISA. The first 20% cells were transferred into a 24-well plate. After 7 days of growth, the antibody yield of the cells was determined, and the first 5 to 6 strains were selected and transferred into a shake flask for culture, thereby achieving the expression of anti-HER2 antibody in CHO-K1 cells. After the serum-free culture was stable, the cells were continuously cultured for 7 days, and then collected for purification and follow-up experiments.

### (10) Construction of expression vector of anti-VEGF antibody Fab fragment and mutant thereof

The DNA encoding the anti-VEGF antibody Fab fragment described herein (the corresponding wild-type sequence was obtained from USP Medicines Compendium, specially the heavy chain sequence of Fab fragment is shown in SEQ ID NO: 25 and the light chain sequence of Fab fragment is shown in SEQ ID NO: 26; based on this, according to kabat numbering, the amino acid at position 166 of the light chain of Fab fragment was substituted with cysteine to obtain a variant 166C of the present invention, and the amino acid at position 124 of the light chain of Fab fragment was substituted with cysteine to obtain a control antibody 124C) was chemically synthesized (Suzhou GENEWIZ Biotechnology Co., Ltd.), then the heavy chain gene of Fab fragment was digested with BstBI and PacI, and the light chain gene of Fab fragment was digested with HindIII and EcoRI; T4 ligase was used to ligate the heavy chain gene of Fab fragment into an eukaryotic expression vector pCGS3 (Biovector NTCC Inc.) treated by the BstBI and PacI; after successful ligation, the expression vector pCGS3 was digested with HindIII and EcoRI, and then the light chain gene of Fab fragment was ligated to the expression vector pCGS3 by T4 ligase, that is, an expression vector containing both heavy and light chains was successfully constructed.

| | WT Fab (Bevacizumab) | 166C | 124C |
|---|---|---|---|
| Encoding Sequence of Heavy Chain of Fab Fragment | SEQ ID NO:27 | SEQ ID NO:27 | SEQ ID NO:27 |
| Encoding Sequence of Light Chain of Fab Fragment | SEQ ID NO:28 | SEQ ID NO:30 | SEQ ID NO:32 |
| Amino Acid Sequence of Heavy Chain of Fab Fragment | SEQ ID NO:25 | SEQ ID NO:25 | SEQ ID NO:25 |
| Amino Acid Sequence of Light Chain of Fab Fragment | SEQ ID NO:26 | SEQ ID NO:29 | SEQ ID NO:31 |

### (11) Screening and purification of expression vector of anti-VEGF antibody Fab fragment and mutant thereof

The successfully constructed expression vector was transformed into DH5α competent *E. coli* strain. The transformed DH5α strain was digested with BstBI and PacI, as well as HindIII and EcoRI respectively, and was verified by sequencing, and then positive clones were selected. A plasmid extraction kit (purchased from CoWin Biosciences) was used to lyse the resulting *E. coli* and extract the plasmid, thereby obtaining an expression vector containing heavy and light chains.

### (12) Expression of anti-VEGF antibody Fab fragment and mutant thereof in CHO cells

The purified expression vector was transfected into CHO-K1 (purchased from ATCC) cells by electroporation; the cells were plated in a 96-well plate, grown for 15 days, and then single clones were picked out, and the antibody yield of the cells was determined by SDS-PAGE. The first 20% cells were transferred into a 24-well plate. After 7 days of growth, the antibody yield of the cells was determined, and the first 5 to 6 strains were selected and transferred into a shake flask for culture, thereby achieving the expression of antibody in CHO-K1 cells. After the serum-free culture was stable, the cells were continuously cultured for 7 days, and then collected for purification via Ni affinity chromatography column and follow-up experiments.

### Example 2 Purification by Pro A affinity chromatography column (HiTrap FF)

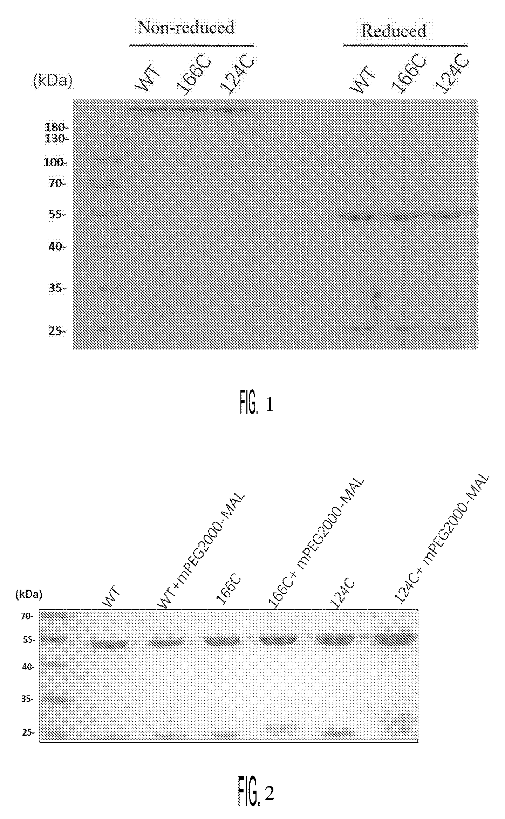

The cells and culture medium were transferred into a 50 mL centrifuge tube, placed in a high-speed refrigerated centrifuge, and centrifuged at 8000 r for 15 min; the supernatant was kept, and the cell debris was discarded; the culture medium was filtered once with a 0.22 µm microfiltration membrane, and the filtrate was collected for later use; the pump, injector, and purification column etc. were washed with 10 column volumes of ultrapure water and PBS; the sample was loaded and eluted with 0.1 M Gly-HCl and the purified antibody protein solution was taken into a EP tube, 1.5 M Tris-HCl was added to adjust the pH to be 7 to 8, and the purified antibody was stored at 2-8 °C. The results are shown in Figure 1.

### Example 3 Detecting the content of free sulfhydryl groups in the antibody

Ellman's method (Thermo Fisher Scientific (China) Co., Ltd.) was used to detect the free sulfhydryl content of the antibody product, and a cell line suitable for further coupling was selected. 0.1 M Na₂HPO₄ solution, DTNB (10 mM) solution, and cysteine standard solution with a final concentration of 0.0059 M to 0.375 M were prepared. At the same time, an antibody sample to be tested was prepared with a concentration of 5 mg/mL. 250 µL 0.1 M Na₂HPO₄ and 5 µL DTNB solution were respectively added into the 1.5 mL EP tube which contains the cysteine standard solution and the sample to be tested, and then the EP tubes were vortexed and shaken for completely mixing, and incubated for 5 min at room temperature in dark. The OD value at 412 nm was detected. A standard curve was established based on the OD value, and the sulfhydryl content of the sample was calculated.

**Table 1. Ellman's method was used to detect the free sulfhydryl content of anti-VEGF antibody products (WT, 166C and 124C); wherein, WT substantially does not contain free sulfhydryl groups, while about 100% of sulfhydryl groups in 166C and 124C are free.**

| **Sample** | **Free Sulfhydryl %** |
|---|---|
| WT | 8.05% |
| 166C | 106.14% |
| 124C | 122.78% |

**Table 2. Ellman's method was used to detect the free sulfhydryl content of anti-CD20 antibody products (WT, 166C and 124C); wherein, WT substantially does not contain free sulfhydryl groups, while about 100% of sulfhydryl groups in 166C and 124C are free.**

| **Sample** | **Free Sulfhydryl %** |
|---|---|
| WT | 10.21% |
| 166C | 100.32% |
| 124C | 98.98% |

**Table 3. Ellman's method was used to detect the free sulfhydryl content of anti-HER2 antibody products (WT, 166C and 124C); wherein, WT substantially does not contain free sulfhydryl groups, while about 100% of sulfhydryl groups in 166C and 124C are free.**

| **Sample** | **Free Sulfhydryl %** |
|---|---|
| WT | 6.11% |
| 166C | 110.28% |
| 124C | 105.38% |

**Table 4. Ellman's method was used to detect the free sulfhydryl content of anti-VEGF antibody Fab fragment products (WT, 166C and 124C); wherein, WT substantially does not contain free sulfhydryl groups, while about 50% of sulfhydryl groups in 166C and 124C are free.**

| **Sample** | **Free Sulfhydryl %** |
|---|---|
| WT | 3.22% |
| 166C | 60.68% |
| 124C | 40.59% |

### Example 4 Antibody conjugates and the detection thereof

The conditions for coupling reaction of antibody and conjugate were as follows: the molar ratio of antibody to conjugate (polypeptide or mPEG2000-MAL) was 1:30, at 37 °C for 3 hour to 4 hour, with a reaction buffer of PBS (pH7.2). The SDS-PAGE electrophoretogram for the coupling of anti-VEGF antibody and mPEG2000-MAL (purchased from a chemical reagent company, such as Shanghai ZZBio Co., Ltd., cat. no.: ZZP-MPEG-MAL-2K-01) was shown in Figure 2.

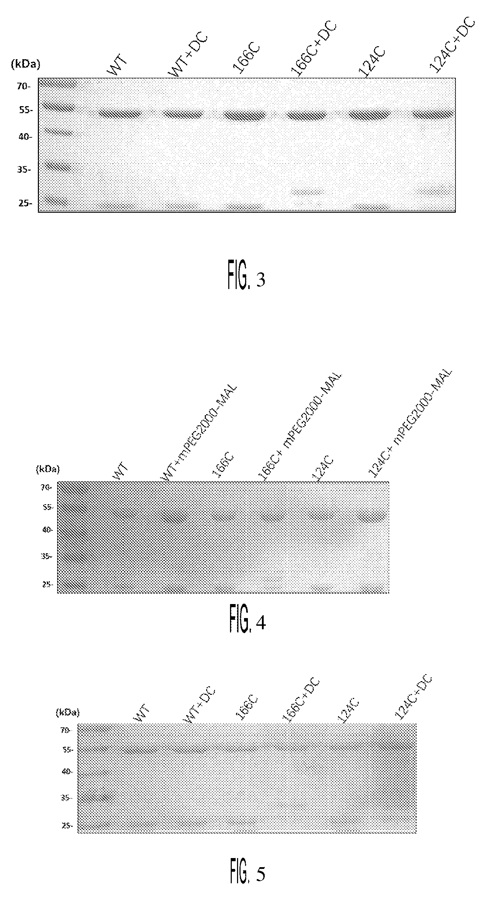

The SDS-PAGE electrophoretogram for the coupling of anti-VEGF antibody and DC (polypeptide-linker) (see formula II below for the reaction, wherein 166ADC was obtained by coupling 166C mutant to DC, and 124ADC was obtained by coupling 124C mutant to DC) was shown in Figure 3. As can be seen by Image J (NIH) grayscale analysis, 85% of 166C was successfully coupled to DC, while only 75% of 124C was successfully coupled to DC. It can be seen that under the same condition, the 166C antibody mutant showed better conjugation efficiency. The construction of DC (polypeptide-linker) was shown as follows:

The polypeptide was an active polypeptide with Ang2 neutralization effect, with a sequence of Gln-Lys(Ac)-Tyr-Gln-Pro-Leu-Asp-Glu-Lys(Ac)-Asp-**Lys**-Thr-Leu-Tyr-Asp-Gln-Phe-Met-Leu-Gln-Gln-Gly-CONH₂(SEQ ID NO:9), and was obtained from Hanhua Huang, Jing-Yu Lai, Janet Do, Dingguo Liu, Lingna Li, Joselyn Del Rosario. Specifically Targeting Angiopoietin-2 Inhibits Angiogenesis, Tie2-Expressing Monocyte Infiltration, and Tumor Growth. Clin Cancer Res; 17(5) March 1, 2011, 1001-1011, wherein the underlined Lys was used to be linked to the linker. The linker was formamide-PEG4-NHS, and the structural formula thereof was shown in formula I below:

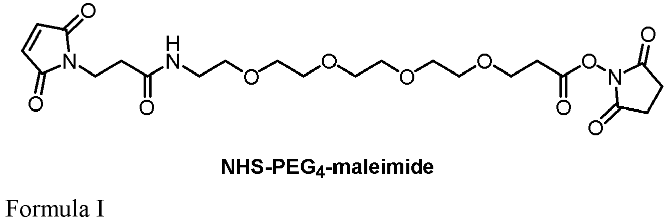

The reaction route of active peptide to linker was as follows

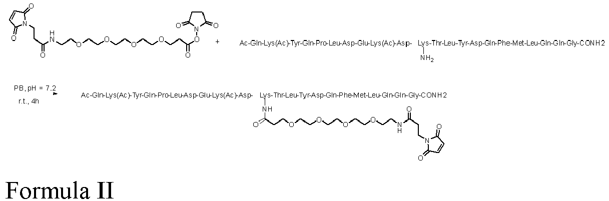

The result for the coupling of anti-CD20 antibody (WT, 124C, 166C) to mPEG2000-MAL was shown in Figure 4;

The result for the coupling of anti-CD20 antibody (WT, 124C, 166C) to DC (polypeptide-linker) was shown in Figure 5.

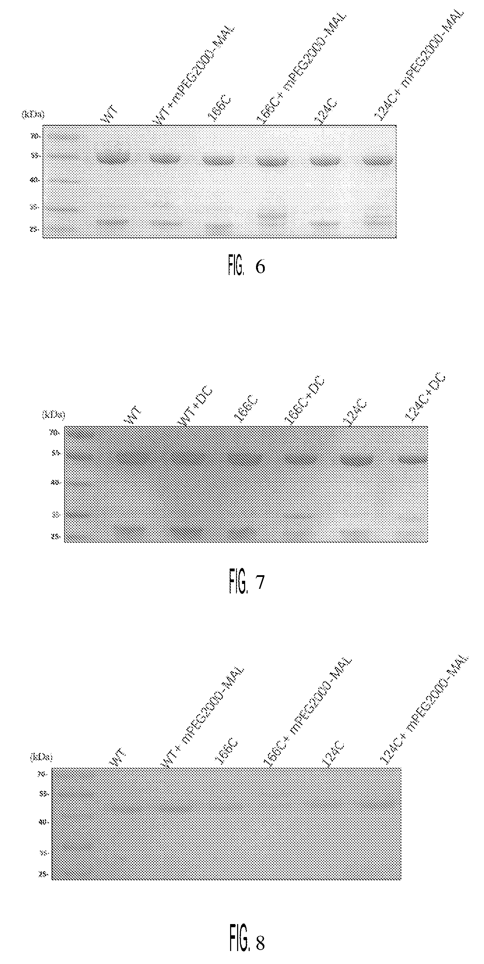

The result for the coupling of anti-HER2 antibody (WT, 124C, 166C) to mPEG2000-MAL was shown in Figure 6;

The result for the coupling of anti-HER2 antibody (WT, 124C, 166C) to DC (polypeptide-linker) was shown in Figure 7.

The result for the coupling of anti-VEGF Fab antibody (WT, 124C, 166C) to mPEG2000-MAL was shown in Figure 8;

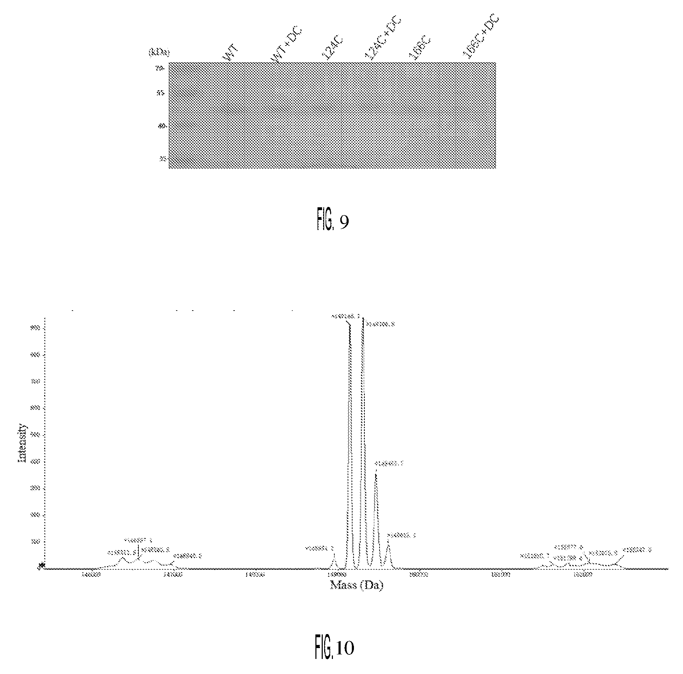

The result for the coupling of anti-VEGF Fab antibody (WT, 124C, 166C) to DC (polypeptide-linker) was shown in Figure 9.

### Mass Spectrometry:

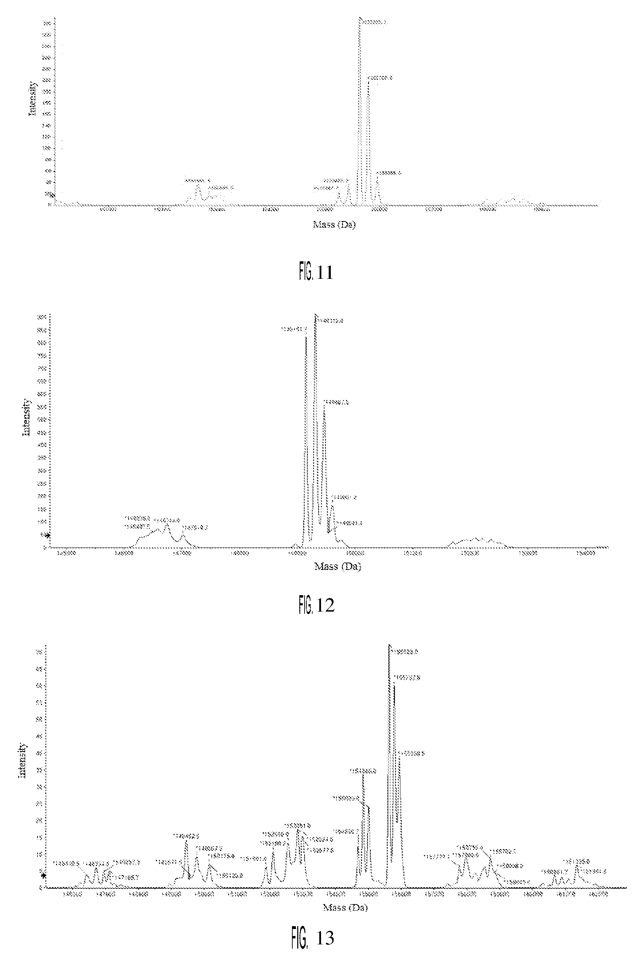

The sample was pre-treated by an ultrafiltration tub and dissolved in double distilled water. The obtained samples were analyzed by mass spectrometry. The mass spectrometry system was a high-resolution tandem mass spectrometry Triple TOF 4600 LC/MS/MS system of AB Sciex, with electrospray ionization source, and a positive ion mode was used for analysis. Mass spectrometry parameters were as follows: GS1: 55; GS2: 55; CUR: 35; scanning range: 100-2000 Da; Ionspray Voltage (ISVF): 5500 V; ionspray temperature: 350 °C; declustering voltage (DP): 150 V; collision energy (CE): 10 eV. The results were shown in Figure 10 to Figure 13. It can be seen from Figure 10 and Figure 11 that the main peak of mutant 166C was shifted to the right by 6478.8 Da after coupling with DC, which was the molecular weight of two molecules of DC. It can be known that both the two sulfhydryl groups on 166C were successfully coupled with DC.

It can be seen from Figure 12 to Figure 13 that after coupling with DC, the mutant 124C generated peaks of coupling with zero molecule DC, peaks of coupling with one molecule DC and peaks of coupling with two molecules DC, and thus the coupling uniformity and efficiency were not as high as that of 166C.

### Example 5 Determination of affinity of anti-VEGF antibody to antigen

The affinity of wild-type antibody, antibody mutants and antibody-polypeptide conjugates to VEGF was determined by Biolayer interferometry (BLI) using Pro A probe (ForteBio). Antibody samples were dissolved in PBS with a working concentration of 10 µg/mL; VEGF-165 (Beijing SinoBiological Co., Ltd.) was dissolved in PBS and gradiently diluted to concentrations of 50 nM, 75 nM, 100 nM, 150 nM, 200 nM respectively. The working volume was 200 µL.

The affinity of wild-type antibody, antibody mutants and antibody-polypeptide conjugates to Ang2 was determined by Biolayer interferometry (BLI) using Pro A probe (ForteBio). Antibody samples were dissolved in PBS with a working concentration of 20 µg/mL; Ang2 (Beijing SinoBiological Co., Ltd.) was dissolved in PBS (0.02% tween-20, 0.1% BSA) and gradiently diluted to concentrations of 25 nM, 50 nM, 75 nM, 100 nM, 200 nM respectively. The working volume was 200 µL.

The data diagram was fitted by OCTET system and data-processing software, and the intermolecular interaction between the antibody and the VEGF or Ang2 was calculated by the software, and shown in K_{D} value. The results are shown in Table 5.

Table 5 shows that there is almost no difference in the binding interaction between the wild-type antibody, antibody mutants and conjugates to the VEGF-165, and the binding interaction of the conjugate to the Ang2 is also within a reasonable range of interaction of neutralization effect.

**Table 5 Detection of sample activity**

| **Sample** | **K_{D} of Antibody to Antigen (10⁻⁹)** | |
|---|---|---|
| | VEGF-165 | Ang2 |
| WT | 1.65 | N.A. |
| 166 | 1.51 | N.A. |
| 124 | 0.99 | N.A. |
| 166ADC | 1.96 | 2.20 |
| 124ADC | 0.87 | 4.64 |

### Example 7 Determining the activity of anti-VEGF antibody on cell proliferation

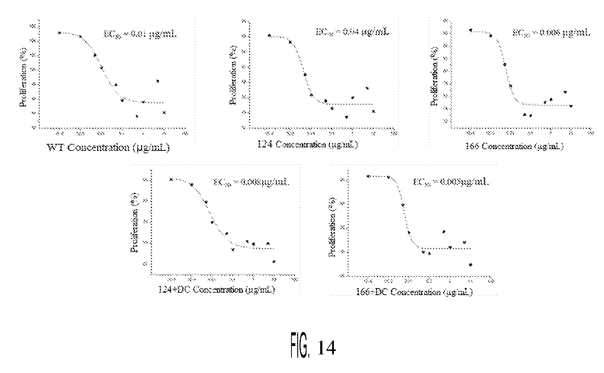

The neutralization effect of antibody on VEGF165 was detected on HUVEC cells (ScienCell Research Laboratories, Inc.). Cells were seeded in a 96-well plate at a density of 4000 cells/50 µL/well. Blank control wells were set up, with no less than 6 control wells. Antibody samples with gradient concentrations (0.001 µg/mL, 0.01 µg/mL, 0.05 µg/mL, 0.1 µg/mL, 0.5 µg/mL, 1 µg/mL, 5 µg/mL, 10 µg/mL) were prepared by a gradient dilution method. VEGF165 was diluted with a test medium to a concentration of 110 ng/mL. The prepared VEGF165 reagent was mixed with samples at gradient concentration to a final concentration of VEGF165 of 10 ng/mL. The 96-well plate was placed in a CO₂ incubator for 48 hours, and then subjected to a CCK-8 counting. The proliferation rate was calculated based on the OD value of the sample and the control. The results are shown in Figure 14, indicating the inhibition of wild-type antibody (WT), mutants (124C and 166C) and conjugates (124ADC and 166ADC) on the proliferation of endothelial cells HUVEC (proliferation produced by VEGF stimulation). It can be known from the figure that the activity of antibody mutant was not changed due to the cysteine mutation at the selected site, indicating that the biological activity of the antibody was not affected by the selected sites. Similarly, the conjugate produced by conjugating with DC has no effect on the activity of the antibody to inhibit cell proliferation.

### Example 8 Determination of anti-CD20 antibody activity

The affinity of wild-type antibody, antibody mutants to CD20 was determined by Biolayer interferometry (BLI) using Pro A probe (ForteBio). Antibody samples were dissolved in PBS with a working concentration of 10 µg/mL; CD20 (Beijing SinoBiological Co., Ltd.) was dissolved in PBS and gradiently diluted to concentrations of 25 nM, 50 nM, 75 nM, 100 nM, 150 nM, 200 nM respectively. The working volume was 200 µL.

The data diagram was fitted by OCTET system and data-processing software, and the intermolecular interaction between the antibody and the CD20 was calculated by the software, and shown in K_{D} value. The results are shown in Table 6.

**Table 6.The affinity of wild-type antibody and antibody mutants to CD20**

| **Sample** | **K_{D} of Antibody to Antigen (10⁻⁹)** |
|---|---|
| WT | 5.35 |
| 166C | 4.86 |
| 124C | 8.97 |

### Example 9 Determination of anti-HER2 antibody activity

The affinity of wild-type antibody, antibody mutants and antibody-polypeptide conjugates to HER-FC was determined by Biolayer interferometry (BLI) using Pro A probe (ForteBio). HER-FC (Beijing SinoBiological Co., Ltd.) was dissolved in PBS with a working concentration of 10 µg/mL; the antibody was dissolved in PBS2 and gradiently diluted to concentrations of 25 nM, 50 nM, 75 nM, 150 nM, 300 nM respectively. The working volume was 200 µL.

The data diagram was fitted by OCTET system and data-processing software, and the intermolecular interaction between the antibody and the HER2 was calculated by the software, and shown in K_{D} value. The results are shown in Table 7.

**Table 7.The affinity of wild-type antibody and antibody mutants to HER2**

| **Sample** | **K_{D} of Antibody to Antigen (10⁻⁹)** |
|---|---|
| WT | 3.58 |
| 166C | 6.87 |
| 124C | 5.46 |

### References

1. Doppalapudi VR, Huang J, Liu D, Jin P, Liu B, Li L, Chemical generation cf bispecific antibodies. Proc Natl Acad Sci U S A. (2010) 107:22611-22616.
2. Allen, T. M., Ligand-targeted therapeutics in anticancer therapy, Nat. Rev. Cancer, (2002) 2:750-763.
3. Hamblett, K. J., Effects of Drug Loading on the Antitumor Activity of a Monoclonal Antibody Drug Conjugate, Clin. Cancer Res.,(2004) 10:7063-7070.
4. Junutula J R, Bhakta S, Raab H, et al. Rapid identifi cation of reactive cysteine residues for site-specific labeling of antibody-Fabs. J Immunol Methods, (2008) 332: 41-52.
5. Junutula J R, Flagella K M, Graham R A, et al. Engineered thiotrastuzumab- DM1 conjugate with an improved therapeutic index to target human epidermal growth factor receptor 2-positive breast cancer. Clin Cancer Res, (2010) 16: 4769-4778.
6. Junutula J R, Raab H, Clark S, et al. Site-specific conjugation of a cytotoxic drug to an antibody improves the therapeutic index. Nat Biotechnol, (2008) 26: 925-932.
7. Kung Sutherland M S, Walter R B, Jeffrey S C, et al. SGN-CD33A: a novel CD33-targeting antibody-drug conjugate using a pyrrolobenzodiazepine dimer is active in models of drug-resistant AML. Blood, (2013) 122: 1455-1463.

### Sequences

SEQ ID NO:1 
EVQLVESGGGLVQPGGSLRLSCAASGYTFTNYGMNWVRQAPGKGLEWVGWINTYTGEP
TYAADFKRRFTFSLDTSKSTAYLQMNSLRAEDTAVYYCAKYPHYYGSSHWYFDVWGQGT
LVTVSSASTKGPSVFPLAPSSKSTSGGTAALGCLVKDYFPEPVTVSWNSGALTSGVHTFPA
VLQSSGLSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDKS
RWQQGNVFSCSVMHEALHNHYTQKSLSLSPGK
SEQ ID NO:2 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:3 
gaagtgcagctggtggaatcaggaggaggactggtgcagccaggaggatctctgagactgtcttgtgctgcttccggatacacctttaccaac
tacggaatgaattgggtgagacaggctccaggaaaaggactggagtgggtgggttggattaacacctataccggagagcctacatacgctg
ccgatttcaagagaaggttcaccttcagcctggatacctctaagtctaccgcttacctgcagatgaactctctgagagcagaggataccgccgt
gtattattgcgctaagtaccctcactactacggctcctcccattggtactttgacgtctggggacagggaacactggtgacagtgtcttccgcttct
acaaaaggaccttccgtgttccctctggctccttcttctaagtctacctcaggaggaacagcagctctgggttgtctggtgaaggattacttccca
gagccagtgacagtgtcttggaactccggagctctgacatccggagtgcatacatttccagcagtgctccagtcttccggactgtattctctgtct
tccgtggtgacagtgccttcttcttctctgggaacacagacctacatctgcaacgtgaaccacaagccttccaacaccaaggtggataagaagg
tggagcctaagtcttgcgacaagacccatacttgtcctccttgtccagctccagaactgctgggaggaccatcagtgtttctgtttcctcctaagc
ctaaggacaccctgatgatctccagaacaccagaagtgacttgcgtggtggtggatgtgtctcacgaagatccagaggtgaagttcaattggta
cgtggacggagtggaagtgcacaacgctaaaaccaagcctagagaggagcagtacaactccacctatagagtggtgtccgtgctgacagtg
ctgcatcaggattggctgaacggaaaggagtacaagtgcaaggtgtccaacaaagctctgccagctcctatcgagaagacaatctccaaggc
taagggacagcctagagaacctcaggtgtatacactgcctccttctagagaggagatgaccaagaatcaggtgtctctgacttgcctggtgaa
gggcttttacccttctgacatcgcagtcgagtgggaatctaacggacagccagagaacaactacaagaccacacctccagtgctggattccga
cggatctttcttcctgtactccaagctgaccgtggataagtctcgttggcagcagggtaacgtgttttcttgtagcgtgatgcacgaagctctgcat
aatcactacacccagaagtctctgtctctgtctcctggaaagtga
SEQ ID NO:4 
gatatccagatgacccagtctccttcctctctgtcagcttcagtgggagatagagtgacaatcacctgttccgcttctcaggatatctccaactacc
tgaattggtaccagcagaagccaggaaaggctcctaaggtgctgatctacttcacctcttctctgcattccggagtgccttctagatttagcggct
ccggctccggcacagatttcacactgaccatctcttctctgcagccagaagatttcgccacctactattgccagcagtactctaccgtgccttgg
acatttggacagggaaccaaggtggagatcaagagaacagtggctgctccttccgtgtttatctttcctccttctgacgaacagctgaaatccgg
aacagcttcagtcgtctgcctgctgaacaacttctaccctagagaggccaaagtccagtggaaagtggataacgctctgcagtccggaaattct
caggaatccgtgaccgagcaggattctaaggattctacctactccctgtcttctaccctgacactgtctaaggccgattacgagaagcacaagg
tgtacgcttgcgaagtgacacatcagggactgtcttctccagtgaccaagtccttcaacagaggcgagtgttga
SEQ ID NO:5 
gatatccagatgacccagtctccttcctctctgtcagcttcagtgggagatagagtgacaatcacctgttccgcttctcaggatatctccaactacc
tgaattggtaccagcagaagccaggaaaggctcctaaggtgctgatctacttcacctcttctctgcattccggagtgccttctagatttagcggct
ccggctccggcacagatttcacactgaccatctcttctctgcagccagaagatttcgccacctactattgccagcagtactctaccgtgccttgg
acatttggacagggaaccaaggtggagatcaagagaacagtggctgctccttccgtgtttatctttcctccttctgacgaacagctgaaatccgg
aacagcttcagtcgtctgcctgctgaacaacttctaccctagagaggccaaagtccagtggaaagtggataacgctctgcagtccggaaattct
caggaatccgtgaccgagtgcgattctaaggattctacctactccctgtcttctaccctgacactgtctaaggccgattacgagaagcacaaggt
gtacgcttgcgaagtgacacatcagggactgtcttctccagtgaccaagtccttcaacagaggcgagtgttga
SEQ ID NO:6 gatatccagatgacccagtctccttcctctctgtcagcttcagtgggagatagagtgacaatcacctgttccgcttctcaggatatctccaactacc 
tgaattggtaccagcagaagccaggaaaggctcctaaggtgctgatctacttcacctcttctctgcattccggagtgccttctagatttagcggct
ccggctccggcacagatttcacactgaccatctcttctctgcagccagaagatttcgccacctactattgccagcagtactctaccgtgccttgg
acatttggacagggaaccaaggtggagatcaagagaacagtggctgctccttccgtgtttatctttcctccttctgacgaatgtctgaaatccgg
aacagcttcagtcgtctgcctgctgaacaacttctaccctagagaggccaaagtccagtggaaagtggataacgctctgcagtccggaaattct
caggaatccgtgaccgagcaggattctaaggattctacctactccctgtcttctaccctgacactgtctaaggccgattacgagaagcacaagg
tgtacgcttgcgaagtgacacatcagggactgtcttctccagtgaccaagtccttcaacagaggcgagtgttga
SEQ ID NO:7 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDEQL
KSGTASVVCLLNNFYPREAKVQWKVDNALQSGNSQESVTECDSKDSTYSLSSTLTLSKAD
YEKHKVYACEVTHQGLSSPVTKSFNRGEC
SEQ ID NO:8 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDECL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:9 
QVQLQQPGAELVKPGASVKMSCKASGYTFTSYNMHWVKQTPGRGLEWIGAIYPGNGDT
SY
NQKFKGKATLTADKSSSTAYMQLSSLTSEDSAVYYCARSTYYGGDWYFNVWGAGTTVTV
S AASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKKAEPKSCDKTHTCPPCPAPELLG
GPSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSR
WQQGNVFSCSVMHEALHNHYTQKSLSLSPGK
SEQ ID NO:10 
QIVLSQSPAILSASPGEKVTMTCRASSSVSYIHWFQQKPGSSPKPWIYATSNLASGVPVRFS
GSGSGTSYSLTISRVEAEDAATYYCQQWTSNPPTFGGGTKLEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:11 
caagtgcaactgcagcagcccggcgccgaactggtgaagcccggcgctagcgtcaagatgagctgtaaggcttccggctacactttcacta
gctacaacatgcactgggtcaagcagacacccggaaggggactggaatggatcggcgctatctaccccggcaacggagacacaagctaca
accaaaagttcaagggcaaggccactctcactgctgataagagcagctccactgcctacatgcaactgagctctctgactagcgaagattccg
ccgtgtactactgcgccagaagcacatactacggcggcgactggtatttcaacgtctggggcgccggcacaacagtgacagtgagcgctgc
cagcactaagggaccatccgtgtttcctctcgccccaagctccaagagcacttccggaggcactgccgctctgggctgtctggtcaaggacta
cttcccagagccagtgacagtcagctggaatagcggagctctgacaagcggcgtccatacattcccagccgtgctgcagtccagcggactgt
actctctgagctccgtcgtgactgtgccaagcagctctctgggcacacagacatacatctgcaatgtcaaccacaagcctagcaacacaaagg
tggacaagaaggccgaaccaaagagctgcgacaagacacacacatgccctccttgtccagccccagagctgctcggaggaccaagcgtct
tcctcttcccacctaagcctaaggacacactgatgatctctaggactccagaggtgacatgcgtcgtcgtcgatgtgtcccacgaggatccaga
ggtgaagttcaactggtacgtggacggcgtcgaagtgcacaatgccaagactaagccaagggaggagcagtacaactccacttacagagtc
gtgtccgtcctcacagtgctccaccaagattggctgaatggcaaggagtataagtgtaaggtctccaataaagctctgccagctcctatcgaga
agactatcagcaaagctaagggccagccaagagagccacaagtgtacactctgcctccatctagggatgagctgactaagaaccaagtgtct
ctgacttgcctcgtgaagggcttctatcctagcgacatcgccgtcgagtgggagtccaacggccaaccagaaaacaactacaagactactcct
ccagtgctcgacagcgatggctccttctttctgtactccaagctgacagtggacaagtccagatggcagcaaggcaacgtcttcagctgtagc
gtgatgcatgaggctctgcacaaccactacactcagaagtctctgtctctgagccccggcaag
SEQ ID NO:12 
Cagatcgtcctcagccagagcccagccattctgagcgccagccccggcgagaaggtgactatgacatgcagagcctccagctccgtgagc
tacatccactggttccagcagaagcccggctcctcccctaagccttggatctacgctacaagcaatctggcctccggagtcccagtgaggttca
gcggcagcggatccggcacttcctactctctgacaatttctagggtggaggctgaggacgctgccacatactactgccagcagtggactagc
aaccctcctacattcggcggaggcacaaagctggagatcaagaggactgtggccgctcctagcgtgttcatcttccctccttccgatgagcag
ctgaagagcggcactgccagcgtggtctgtctgctgaacaacttctaccctagggaggccaaggtgcaatggaaggtggacaatgctctgca
gtccggcaattcccaagagtccgtcacagagcaagattccaaggacagcacatactccctctccagcacactgacactgagcaaggctgact
acgagaagcacaaggtgtacgcttgtgaggtgactcatcaaggactgagcagcccagtgactaagagcttcaataggggcgagtgt
SEQ ID NO:13 
QIVLSQSPAILSASPGEKVTMTCRASSSVSYIHWFQQKPGSSPKPWIYATSNLASGVPVR
FSGSGSGTSYSLTISRVEAEDAATYYCQQWTSNPPTFGGGTKLEIKRTVAAPSVFIFPPS
DEQLKSGTASVVCLLNNFYPREAKVQWKVDNALQSGNSQESVTECDSKDSTYSLSSTLTL
SKADYEKHKVYACEVTHQGLSSPVTKSFNRGEC
SEQ ID NO:14 
Cagatcgtcctcagccagagcccagccattctgagcgccagccccggcgagaaggtgactatgacatgcagagcctccagctccgtgagc
tacatccactggttccagcagaagcccggctcctcccctaagccttggatctacgctacaagcaatctggcctccggagtcccagtgaggttca
gcggcagcggatccggcacttcctactctctgacaatttctagggtggaggctgaggacgctgccacatactactgccagcagtggactagc
aaccctcctacattcggcggaggcacaaagctggagatcaagaggactgtggccgctcctagcgtgttcatcttccctccttccgatgagcag
ctgaagagcggcactgccagcgtggtctgtctgctgaacaacttctaccctagggaggccaaggtgcaatggaaggtggacaatgctctgca
gtccggcaattcccaagagtccgtcacagagtgtgattccaaggacagcacatactccctctccagcacactgacactgagcaaggctgacta
cgagaagcacaaggtgtacgcttgtgaggtgactcatcaaggactgagcagcccagtgactaagagcttcaatagggggcgagtgt
SEQ ID NO:15 
QIVLSQSPAILSASPGEKVTMTCRASSSVSYIHWFQQKPGSSPKPWIYATSNLASGVPVRFS
GSGSGTSYSLTISRVEAEDAATYYCQQWTSNPPTFGGGTKLEIKRTVAAPSVFIFPPSDECL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:16 
Cagatcgtcctcagccagagcccagccattctgagcgccagccccggcgagaaggtgactatgacatgcagagcctccagctccgtgagc
tacatccactggttccagcagaagcccggctcctcccctaagccttggatctacgctacaagcaatctggcctccggagtcccagtgaggttca
gcggcagcggatccggcacttcctactctctgacaatttctagggtggaggctgaggacgctgccacatactactgccagcagtggactagc
aaccctcctacattcggcggaggcacaaagctggagatcaagaggactgtggccgctcctagcgtgttcatcttccctccttccgatgagtgtc
tgaagagcggcactgccagcgtggtctgtctgctgaacaacttctaccctagggaggccaaggtgcaatggaaggtggacaatgctctgcag
tccggcaattcccaagagtccgtcacagagcaagattccaaggacagcacatactccctctccagcacactgacactgagcaaggctgacta
cgagaagcacaaggtgtacgcttgtgaggtgactcatcaaggactgagcagcccagtgactaagagcttcaataggggcgagtgt
SEQ ID NO:17 
EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGP
SVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK
SEQ ID NO:18 
gaagtgcaactggtggagtccggaggcggactggtccaacccggcggctctctgagactgagctgtgctgccagcggcttcaacatcaagg
acacttacatccattgggtgaggcaagcccccggcaaaggactggaatgggtggctaggatctacccaactaacggctacactaggtatgcc
gacagcgtcaagggaaggttcactatctccgccgacacaagcaagaacactgcctatctgcagatgaactctctgagggctgaggacacag
ctgtgtattactgctctaggtggggcggagatggcttctacgctatggactactggggacaaggcacactcgtgacagtgagcagcgctagca
 
ctaaaggcccttccgtgttcccactcgcccctagctccaaaagcactagcggcggaacagctgctctgggctgtctcgtgaaggactatttccc
agagccagtgactgtgagctggaatagcggcgctctgacaagcggagtgcacacttttccagccgtgctgcagagcagcggactgtattctc
tgtccagcgtcgtcactgtgcctagctcctctctgggcacacagacttacatctgtaacgtgaatcataagccaagcaacactaaggtcgacaa
gaaggtcgagccaaagagctgtgacaagacacacacatgcccaccatgtccagccccagagctcctcggaggacctagcgtgttcctcttcc
ctcctaagccaaaggacactctcatgatctctaggacaccagaggtcacttgcgtggtggtcgatgtcagccacgaggacccagaggtgaag
ttcaattggtacgtggacggcgtggaggtgcataatgccaagacaaagcctagggaggagcagtacaacagcacttacagagtggtctccgt
cctcacagtgctgcaccaagattggctcaacggcaaggagtacaagtgcaaggtgtccaacaaggccctcccagcccctatcgagaagact
atctccaaggccaagggccagccaagagaacctcaagtgtacacactgcctccatctagggaggagatgacaaagaaccaagtgtctctga
catgtctggtgaaaggcttctacccaagcgacatcgccgtggagtgggaaagcaacggccagccagagaacaactacaagactacacctcc
agtgctggactccgacggcagcttcttcctctactccaagctcactgtggataagtctaggtggcagcaaggcaatgtgttcagctgttccgtga
tgcacgaggctctgcacaaccactacactcagaagtctctgagcctctcccccggcaaa
SEQ ID NO:19 
DIQMTQSPSSLSASVGDRVTITCRASQDVNTAVAWYQQKPGKAPKLLIYSASFLYSGVPSR
FSGSRSGTDFTLTISSLQPEDFATYYCQQHYTTPPTFGQGTKVEIKRTVAAPSVFIFPPSDEQ
LKSGTASVVCLLNNFYPREAKVQWKVDNALQSGNSQESVTEQDSKDSTYSLSSTLT
LSKADYEKHKVYACEVTHQGLSSPVTKSFNRGEC
SEQ ID NO:20 
Gacatccagatgacacagtccccttcctctctgagcgctagcgtcggcgatagggtgacaatcacttgtagggccagccaagatgtcaacac
agccgtggcttggtaccagcagaagcccggcaaggcccctaagctgctcatctatagcgccagctttctgtacagcggcgtccctagcagatt
ctccggaagcagatccggcactgacttcacactgactatcagcagcctccagccagaggatttcgccacatactactgccagcagcactaca
ctactccacctactttcggccaaggcactaaggtggagattaagaggactgtcgctgccccaagcgtcttcatcttcccacctagcgatgaaca
gctcaagagcggcacagctagcgtcgtgtgtctgctgaacaacttctaccctagggaggccaaggtccagtggaaagtggataacgctctgc
agagcggcaattcccaagagagcgtcacagagcaagatagcaaagacagcacttactctctgagcagcacactgactctgtccaaggccga
ctacgagaagcacaaggtctatgcttgtgaggtcactcaccaaggactgtccagcccagtcactaagagcttcaataggggcgagtgc
SEQ ID NO:21 
DIQMTQSPSSLSASVGDRVTITCRASQDVNTAVAWYQQKPGKAPKLLIYSASFLYSGVPSR
FSGSRSGTDFTLTISSLQPEDFATYYCQQHYTTPPTFGQGTKVEIKRTVAAPSVFIFPPSDEQ
LKSGTASVVCLLNNFYPREAKVQWKVDNALQSGNSQESVTECDSKDSTYSLSSTLT
LSKADYEKHKVYACEVTHQGLSSPVTKSFNRGEC
SEQ ID NO:22 
Gacatccagatgacacagtccccttcctctctgagcgctagcgtcggcgatagggtgacaatcacttgtagggccagccaagatgtcaacac
agccgtggcttggtaccagcagaagcccggcaaggcccctaagctgctcatctatagcgccagctttctgtacagcggcgtccctagcagatt
ctccggaagcagatccggcactgacttcacactgactatcagcagcctccagccagaggatttcgccacatactactgccagcagcactaca
ctactccacctactttcggccaaggcactaaggtggagattaagaggactgtcgctgccccaagcgtcttcatcttcccacctagcgatgaaca
gctcaagagcggcacagctagcgtcgtgtgtctgctgaacaacttctaccctagggaggccaaggtccagtggaaagtggataacgctctgc
agagcggcaattcccaagagagcgtcacagagtgcgatagcaaagacagcacttactctctgagcagcacactgactctgtccaaggccga
ctacgagaagcacaaggtctatgcttgtgaggtcactcaccaaggactgtccagcccagtcactaagagcttcaataggggcgagtgc
SEQ ID NO:23 
DIQMTQSPSSLSASVGDRVTITCRASQDVNTAVAWYQQKPGKAPKLLIYSASFLYSGVPSR
FSGSRSGTDFTLTISSLQPEDFATYYCQQHYTTPPTFGQGTKVEIKRTVAAPSVFIFPPSDECL
KSGTASVVCLLNNFYPREAKVQWKVDNALQSGNSQESVTEQDSKDSTYSLSSTLT
LSKADYEKHKVYACEVTHQGLSSPVTKSFNRGEC
SEQ ID NO:24 
Gacatccagatgacacagtccccttcctctctgagcgctagcgtcggcgatagggtgacaatcacttgtagggccagccaagatgtcaacac
agccgtggcttggtaccagcagaagcccggcaaggcccctaagctgctcatctatagcgccagctttctgtacagcggcgtccctagcagatt
ctccggaagcagatccggcactgacttcacactgactatcagcagcctccagccagaggatttcgccacatactactgccagcagcactaca
ctactccacctactttcggccaaggcactaaggtggagattaagaggactgtcgctgccccaagcgtcttcatcttcccacctagcgatgaatg
cctcaagagcggcacagctagcgtcgtgtgtctgctgaacaacttctaccctagggaggccaaggtccagtggaaagtggataacgctctgc
 
agagcggcaattcccaagagagcgtcacagagcaagatagcaaagacagcacttactctctgagcagcacactgactctgtccaaggccga
ctacgagaagcacaaggtctatgcttgtgaggtcactcaccaaggactgtccagcccagtcactaagagcttcaataggggcgagtgc
SEQ ID NO:25 
EVQLVESGGGLVQPGGSLRLSCAASGYTFTNYGMNWVRQAPGKGLEWVGWINTYTGEP
TYAADFKRRFTFSLDTSKSTAYLQMNSLRAEDTAVYYCAKYPHYYGSSHWYFDVWGQGT
LVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHT
SEQ ID NO:26 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:27 
Gaggtgcaactggtcgaaagcggaggcggactggtgcagcccggcggatctctgaggctgagctgtgccgccagcggctacactttcact
aactacggcatgaattgggtgaggcaagcccccggcaaaggactggagtgggtcggatggatcaacacttacactggcgagccaacttacg
ccgccgacttcaagaggaggttcacattcagcctcgacactagcaagagcacagcctatctgcagatgaacagcctcagagctgaggacact
gccgtgtactactgcgctaagtatcctcactactacggcagcagccactggtatttcgatgtgtggggccaaggcacactcgtcactgtcagca
gcgcttccacaaaaggccctagcgtgttccctctcgcccctagctccaagagcacatccggaggaacagctgctctcggctgtctggtgaag
gattacttcccagagccagtgactgtcagctggaatagcggcgctctgacaagcggcgtgcacacattcccagccgtgctgcagagctccgg
actgtactccctcagcagcgtggtcacagtccctagcagctctctgggcactcagacatacatctgcaacgtgaaccacaagccaagcaaca
ctaaggtcgacaagaaggtggagcctaagagctgcgacaagactcacaca
SEQ ID NO:28 
Gatatccagatgacccagtctccttcctctctgtcagcttcagtgggagatagagtgacaatcacctgttccgcttctcaggatatctccaactac
ctgaattggtaccagcagaagccaggaaaggctcctaaggtgctgatctacttcacctcttctctgcattccggagtgccttctagatttagcggc
tccggctccggcacagatttcacactgaccatctcttctctgcagccagaagatttcgccacctactattgccagcagtactctaccgtgccttgg
acatttggacagggaaccaaggtggagatcaagagaacagtggctgctccttccgtgtttatctttcctccttctgacgaacagctgaaatccgg
aacagcttcagtcgtctgcctgctgaacaacttctaccctagagaggccaaagtccagtggaaagtggataacgctctgcagtccggaaattct
caggaatccgtgaccgagcaggattctaaggattctacctactccctgtcttctaccctgacactgtctaaggccgattacgagaagcacaagg
tgtacgcttgcgaagtgacacatcagggactgtcttctccagtgaccaagtccttcaacagaggcgagtgt
SEQ ID NO:29 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDEQL
KSGTASVVCLLNNFYPREAKVQWKVDNALQSGNSQESVTECDSKDSTYSLSSTLTLSKAD
YEKHKVYACEVTHQGLSSPVTKSFNRGEC
SEQ ID NO:30 
gatatccagatgacacagtccccttcctctctgagcgctagcgtgggcgatagggtgacaattacttgtagcgccagccaagatatcagcaact
atctgaactggtaccagcagaagcccggcaaggccccaaaggtcctcatctacttcactagcagcctccacagcggcgtcccatccagattta
gcggcagcggcagcggaactgacttcacactgacaatcagctctctgcagccagaggatttcgccacttactactgccagcagtactccaca
gtcccttggacatttggccaaggcactaaggtggagatcaagagaacagtggccgccccctagcgtcttcatcttcccaccttccgatgagcag
ctgaagagcggaacagcttccgtggtctgtctgctgaacaacttctacccaagagaggccaaggtccagtggaaagtggacaacgctctgca
gagcggcaattcccaagagtccgtcacagagtgcgattccaaggacagcacatactctctgagcagcacactgactctgagcaaggccgac
tacgagaagcacaaggtctacgcttgtgaggtcactcaccaaggactgagcagcccagtgactaagtccttcaataggggcgagtgctga
SEQ ID NO:31 
DIQMTQSPSSLSASVGDRVTITCSASQDISNYLNWYQQKPGKAPKVLIYFTSSLHSGVPSRF
SGSGSGTDFTLTISSLQPEDFATYYCQQYSTVPWTFGQGTKVEIKRTVAAPSVFIFPPSDECL
KSGTASVVCLLNNFYPREAKVQWKVDNALQSGNSQESVTEQDSKDSTYSLSSTLTLSKAD
YEKHKVYACEVTHQGLSSPVTKSFNRGEC
SEQ ID NO:32

gatatccagatgacccagtctccttcctctctgtcagcttcagtgggagatagagtgacaatcacctgttccgcttctcaggatatctccaactacc
tgaattggtaccagcagaagccaggaaaggctcctaaggtgctgatctacttcacctcttctctgcattccggagtgccttctagatttagcggct
ccggctccggcacagatttcacactgaccatctcttctctgcagccagaagatttcgccacctactattgccagcagtactctaccgtgccttgg
acatttggacagggaaccaaggtggagatcaagagaacagtggctgctccttccgtgtttatctttcctccttctgacgaatgtctgaaatccgg
aacagcttcagtcgtctgcctgctgaacaacttctaccctagagaggccaaagtccagtggaaagtggataacgctctgcagtccggaaattct
caggaatccgtgaccgagcaggattctaaggattctacctactccctgtcttctaccctgacactgtctaaggccgattacgagaagcacaagg
tgtacgcttgcgaagtgacacatcagggactgtcttctccagtgaccaagtccttcaacagaggcgagtgt

## Claims

1. A variant of antibody or fragment thereof, wherein the antibody or fragment thereof comprises a light chain constant region, and an amino acid at position 166 according to a Kabat numbering system is mutated to cysteine; the antibody preferably is a humanized antibody, a chimeric antibody, more preferably is an IgG antibody.

2. The variant of antibody or fragment thereof of claim 1, wherein the light chain is of a lambda or kappa type.

3. The variant of antibody or fragment thereof of claim 1 or 2, which comprises a heavy chain and a light chain, wherein an amino acid sequence of the heavy chain is shown in SEQ ID NO: 1, and an amino acid sequence of the light chain is shown in SEQ ID NO: 3; the amino acid sequence of the heavy chain is shown in SEQ ID NO: 9, and the amino acid sequence of the light chain is shown in SEQ ID NO: 13; or the amino acid sequence of the heavy chain is shown in SEQ ID NO: 17, and the amino acid sequence of the light chain is shown in SEQ ID NO:21.

4. The variant of antibody or fragment thereof of any one of claims 1 to 3, wherein the fragment is a Fab fragment, a Fab' fragment or a F(ab')₂ fragment, preferably a heavy chain amino acid sequence of the Fab fragment is shown in SEQ ID NO: 25, and a light chain amino acid sequence of the Fab fragment is shown in SEQ ID NO: 29.

5. A bispecific antibody or fusion protein comprising the variant of antibody or fragment thereof of any one of claims 1 to 4.

6. A conjugate comprising the variant of antibody or fragment thereof of any one of claims 1 to 4.

7. The conjugate of claim 6, wherein the conjugate is selected from polyethylene glycol, a cytotoxic agent, an active peptide, a nanobody, a single domain antibody, a Fab fragment, a Fab' fragment, scFv, a small molecule drug, a chemotherapeutic agent or a radiotherapeutic agent; preferably, the active peptide is Agn2 active peptide, more preferably an Agn2 active peptide as shown in SEQ ID NO: 5; more preferably, the Agn2 active peptide is conjugated to a bevacizumab variant of the present invention via a linker; further preferably, the linker has an amide bond, specifically is formamide-PEG₄-NHS, with a structure as shown in formula I

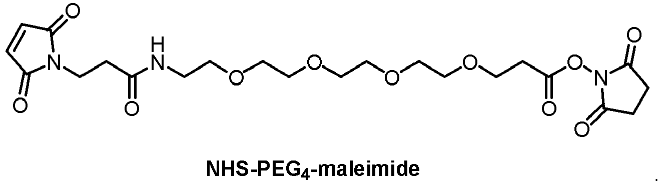

8. The conjugate of claim 6, wherein the polyethylene glycol is monomethoxy polyethylene glycol, preferably mPEG2000, mPEG5000, mPEG10000.

9. The conjugate of claim 7 or 8, wherein the polyethylene glycol is further conjugated to a drug molecule, preferably the polyethylene glycol is a linear difunctionalized polyethylene glycol, a linear heterofunctionalized polyethylene glycol or a multi-arm functionalized polyethylene glycol.

10. A pharmaceutical composition comprising the variant of antibody or fragment thereof of any one of claims 1 to 4, the bispecific antibody or fusion protein of claim 5, the conjugate of any one of claims 6 to 9, and optionally, a pharmaceutically acceptable carrier.

11. A method for treatment of cancer (preferably non-small cell lung cancer such as advanced, metastatic or recurrent non-squamous cell non-small cell lung cancer, colorectal cancer such as metastatic colorectal cancer, breast cancer, malignant glioma and renal cell carcinoma, glioblastoma multiforme), comprising administering the variant of antibody or fragment thereof of any one of claims 1 to 4, the bispecific antibody or fusion protein of claim 5, the conjugate of any one of claims 6 to 9, or the pharmaceutical composition of claim 10.

12. Use of the variant of antibody or fragment thereof of any one of claims 1 to 4, the bispecific antibody or fusion protein of claim 5, or the conjugate of any one of claims 6 to 9, or the pharmaceutical composition of claim 10 in the preparation of a medicament or kit for treatment of cancer (preferably non-small cell lung cancer such as advanced, metastatic or recurrent non-squamous cell non-small cell lung cancer, colorectal cancer such as metastatic colorectal cancer, breast cancer, malignant glioma and renal cell carcinoma, glioblastoma multiforme).

13. A kit comprising the variant of antibody or fragment thereof of any one of claims 1 to 4, the bispecific antibody/fusion protein of claim 5, or the conjugate of any one of claims 6 to 9, or the pharmaceutical composition of claim 10, preferably, the kit further comprises an agent to be administered in combination with the antibody or fragment thereof, such as carboplatin or cisplatin.
